# EUROPEAN PATENT APPLICATION

(11) **EP 4 628 570 A1**
(43) Date of publication of application: **08.10.2025**
(21) Application number: 24168235.0
(22) Date of filing: 03.04.2024
(51) Int. Cl.: C12N 5/00, C12N 5/078

(54) **METHOD FOR CULTIVATING MAMMALIAN CELLS**

(71) Applicant: Aposcience AG, 1200 Wien (AT)
(72) Inventor: Ankersmit, Jan Hendrik, 1060 Wien (AT); Gugerell, Alfred, 1200 Wien (AT); Mildner, Michael, 3040 Neulengbach (AT)
(74) Representative: Schwarz & Partner Patentanwälte GmbH

(57) **Abstract**

The present invention relates to a method for cultivating mammalian cells comprising the step of incubating mammalian cells in a culture medium comprising at least one sodium salt, at least one potassium salt and at least calcium salt under an atmosphere comprising less than 1% carbon dioxide.

## Description

### TECHNICAL FIELD

The present invention relates to the field of mammalian cell cultures.

### BACKGROUND ART

Mammalian cells are typically cultivated in cell culture media in the presence of an atmosphere comprising carbon dioxide (CO₂). Carbon dioxide helps maintain the pH of the culture medium within a suitable range for mammalian cell. When CO₂ dissolves in the culture medium, it forms carbonic acid, which acts as a buffer and helps to prevent the medium from becoming too acidic or basic. CO₂ also plays a role in regulating the osmolarity of the culture medium, ensuring that the cells are in an optimal environment. The typical CO₂ concentration when cultivating mammalian cells is around 5%. This level is intended to maintain a pH balance in the culture medium through bicarbonate buffering and facilitate optimal cell metabolism. However, there are some disadvantages of using a CO₂ comprising atmosphere during the cultivation of mammalian cells.

The need for a controlled supply of CO₂, usually through CO₂ incubators, adds to the overall cost of culturing mammalian cells. As fluctuations in concentration or exposure to air during medium changes may negatively influence mammalian cells, stable CO₂ levels have to be assured. In particular mammalian cells are sensitive to changes in pH, and CO₂-driven pH regulation may not provide optimal conditions for all cell types. Some cell lines may require specialized conditions or alternative methods for pH regulation.

Overall, while CO₂ is commonly used for cultivating mammalian cells due to its pH regulation and carbon source properties, its cost, control challenges and potential limitations for certain cell types should be considered when designing cell culture methods.

It is thus an object of the present invention to provide a method for cultivating mammalian cells which overcomes the disadvantages of using a CO₂ enriched atmosphere.

### SUMMARY OF THE INVENTION

The present invention relates to a method for cultivating mammalian cells comprising the step of incubating mammalian cells in a culture medium comprising at least one sodium salt, at least one potassium salt and at least calcium salt under an atmosphere comprising less than 1% carbon dioxide.

It turned surprisingly out that the mammalian cells can be cultivated under an atmosphere comprising less than 1% CO₂ using a cell culture medium comprising at least one sodium salt, at least one potassium salt and at least calcium salt. It is a surprising finding that using said culture medium allows to keep the pH in a range suitable for mammalian cells although the CO₂ concentration in the atmosphere, where the cells are cultivated, is low compared to typically used atmospheres/gases comprising 4 to 7% or approx. 5%, CO₂. This is particularly advantageous since a CO₂ incubator, which is typically used to cultivate mammalian cells, is not needed and even air (i.e. atmospheric air) can be used to cultivate mammalian cells without adding additional CO₂.

Another aspect of the present invention relates to a method for obtaining a supernatant of mammalian, preferably human, cells comprising the steps of cultivating said cells in a culture medium comprising at least one sodium salt, at least one potassium salt and at least calcium salt under an atmosphere comprising less than 1% carbon dioxide and isolating said supernatant by removing the cells. The mammalian cells are preferably peripheral blood mononuclear cell (PBMC) cells, more preferably human PBMCs.

### BRIEF DESCRIPTION OF THE FIGURES

Fig. 1 shows the enhanced potency of the supernatant when cultivated with low CO₂ concentration measured by Hsp27 phosphorylation.
Fig. 2 shows an enhanced, but already comparable potency of the supernatant when cultivated with low CO₂ concentration in a new medium measured by AP1 activation.
Fig. 3 shows comparable concentrations of EGF in supernatants after cultivation with 5% CO₂ using the established medium or lower concentration of CO₂ using a new medium.
Fig. 4 shows comparable concentrations of TGF beta 1 in supernatants after cultivation with 5% CO₂ using the established medium or lower concentration of CO₂ using a new medium.
Fig. 5 shows comparable induction of cell sprouting in murine aortic tissue between the supernatants after cultivation with 5% CO₂ using the established medium or lower concentration of CO₂ using a new medium.

### DESCRIPTION OF EMBODIMENTS

According to the present invention the atmosphere is formed by a gas which is added to the container (e.g. bioreactor) where the culture medium and the mammalian cells are arranged. The gas can be located in or added to the headspace of said container and/or added by using aeration means positioned preferably in the container, preferably bioreactor.

According to a preferred embodiment of the present invention the atmosphere comprises less than 0.5%, preferably less than 0.2%, more preferably less than 0.1%, more preferably less than 0.05%, carbon dioxide.

According to another preferred embodiment of the present invention the atmosphere comprises oxygen and/or nitrogen.

The atmosphere under which the mammalian cells are incubated and cultivated comprises preferably also oxygen, preferably 0.5 to 21%, more preferably 1 to 21%, even more preferably 5 to 21%, even more preferably 10 to 21%, oxygen. Said atmosphere may also comprise nitrogen, preferably 60 to 78%, more preferably 65 zo 78%, more preferably 70 to 78%, nitrogen. In a particular preferred embodiment of the present invention the atmosphere is air.

In order to prevent infections of the cell culture it is preferred to use sterilized gas or air to obtain the atmosphere. Methods for preparing an atmosphere for cell culturing methods are well known to the person skilled in the art and may include filtration, UV light sterilization and ozone treatment, whereby physical methods like filtration are preferred to avoid any residues which may be harmful for the cells to be cultivated. Filtration, for instance, involves passing the air through a filter that can trap bacteria and viruses. UV light sterilization uses ultraviolet light to kill microorganisms. Ozone treatment involves using ozone gas to disinfect the air.

According to a further preferred embodiment of the present invention the culture medium comprises 50 to 200 mmol/L, preferably 100 to 150 mmol/L, more preferably 125 to 135 mmol/L, more preferably 130 to 132 mmol/L, more preferably around 131 mmol/L, sodium ions.

"Around", as used herein, means that the concentration to which the term is associated to can be 10% higher or lower, preferably 5 % higher or lower, more preferably 4 % higher or lower, more preferably 3 % higher or lower, more preferably 2 % higher or lower, more preferably 1 % higher or lower.

According to another preferred embodiment of the present invention the at least one sodium salt is sodium chloride.

According to a preferred embodiment of the present invention the culture medium comprises 2 to 8 mmol/L, preferably 3 to 6 mmol/L, more preferably 4 to 6 mmol/L, more preferably 5 to 6 mmol/L, more preferably around 5.36 mmol/L, potassium ions.

According to another preferred embodiment of the present invention the at least one potassium salt is potassium chloride.

According to a further preferred embodiment of the present invention the culture medium comprises 0.5 to 3 mmol/L, preferably 1 to 2 mmol/L, more preferably 1.5 to 2 mmol/L, more preferably around 1.84 mmol/L, calcium ions.

According to a preferred embodiment of the present invention the at least one calcium salt is calcium chloride.

The culture medium to cultivate the mammalian cells is preferably Ringer's solution (USP43-NF38 (2020), Monograph "Ringer's Injection", S. 3887).

According to another preferred embodiment of the present invention the culture medium comprises further at least one salt of a C₂ to C₅ carbonic acid, wherein the C₂ to C₅ carbonic acid is preferably lactate, more preferably L-lactate.

The addition of one or more carbonic acids or salts thereof to the culture medium serves multiple purposes. Carbonic acids or salts thereof may be metabolized by the mammalian cells as a source of energy during cultivation. They may act as a buffer, helping to maintain the pH of the culture medium within a physiological range. It helps stabilize the pH and prevents it from becoming too acidic or alkaline by serving as a weak acid/base reservoir. Carbonic acids or salts thereof may assist in maintaining the balance of electrolytes in the solution. It is often included in Ringer's solution to prevent destabilization of sodium, potassium and chloride levels. Overall, carbonic acids or salts thereof, in particular lactate, in the culture medium plays an important role in providing energy, maintaining pH balance, and ensuring electrolyte stability in the body.

In a preferred embodiment of the present invention the culture medium comprises 10 to 50 mmol/L, preferably 15 to 40 mmol/L, more preferably 20 to 30 mmol/L, more preferably around 28 mmol/L, of at least one salt of a C₂ to C₅ carbonic acid, preferably at least one salt of lactate.

According to a preferred embodiment of the present invention the at least one salt of a C₂ to C₅ carbonic acid is an alkaline salt or an alkaline earth salt, preferably a sodium salt. Hence, in a particularly preferred embodiment of the present invention the culture medium is lactated Ringer's solution (USP43-NF38 (2020), Monograph "Lactated Ringer's Injection", S. 3891).

According to a further preferred embodiment of the present invention the culture medium comprises at least one polypeptide, preferably at least one blood polypeptide.

According to a preferred embodiment of the present invention the at least one polypeptide is selected from the group consisting of albumin, preferably serum albumin, and insulin.

According to another preferred embodiment of the present invention the culture medium comprises albumin, preferably serum albumin, and/or insulin.

Polypeptides like albumin in the culture medium of the present invention may serve as a colloidal osmotic agent. Albumin is a protein found in blood plasma that helps maintain the balance of fluid in the body by exerting an osmotic pressure that keeps fluid from leaking out of blood vessels. When added to the culture medium, albumin helps maintain the proper fluid balance.

Also the presence of insulin in the cell culture medium of the present invention can be beneficial. Insulin may have an influence on the growth and maintenance of cells in vitro. Insulin is known to bind to insulin receptors on the cell surface, triggering a cascade of signaling pathways that regulate cell growth and survival. These signaling pathways may have an influence on controlling cell cycle progression, protein synthesis and other cellular processes.

According to a further preferred embodiment of the present invention the culture medium comprises 1 mg/L to 50 g/L, preferably 2 mg/L to 40 g/L, more preferably 4 mg/L to 30 g/L, more preferably 1 g/L to 20 g/L, more preferably 2 g/L to 10 g/L, of the at least one polypeptide.

According to a preferred embodiment of the present invention the culture medium comprises 1 to 50 g/L, preferably 2 to 40 g/L, more preferably 4 to 30 g/L, more preferably 5 to 20 g/L, more preferably 6 to 10 g/L, albumin, preferably serum albumin.

According to another preferred embodiment of the present invention the culture medium comprises 1 to 20 mg/L, preferably 2 to 15 mg/L, more preferably 3 to 10 mg/L, more preferably 4 to 6 mg/L, insulin.

According to a preferred embodiment of the present invention the culture medium has a pH of 5 to 7.

According to a further preferred embodiment the mammalian cells are incubated/cultivated in the couture medium of the present invention at a temperature of 30°C to 38°C, preferably 35°C to 38 °C.

According to a particularly preferred embodiment of the present invention the cells are peripheral blood mononuclear cells (PBMCs), more preferably monocytes, T cells, B cells and/or NK cells.

The method of the present invention is particularly suited to cultivate PBMCs, preferably PBMCs subjected to stress, more preferably irradiated PBMCs.

Cells, in particular mammalian cells, are known to secrete numerous substances during cultivation into a cell culture medium. The so obtained conditioned culture media can be used in the treatment and/or prevention of various diseases and disorders. For instance, WO 2010/070105 and WO 2010/079086 disclose conditioned culture media ("supernatants") which are obtained by cultivation of PBMCs and which can be used in the treatment of various inflammatory conditions. Hence, the method of the present invention is preferably used to obtain a supernatant of a PBMC cell culture, where the cells are cultivated in the culture medium of the present invention. After the cultivation step the cultivated PBMCs are removed from the culture medium in order to obtain the substantially cell-free, preferably completely cell-free, supernatant. The supernatant of the PBMC culture comprises next to components of the cultivation medium substances produced and secreted by the PBMCs and/or even lysed PBMCs. "Supernatant" can be used interchangeable with conditioned culture medium or secretome obtainable by cultivating PBMCs.

The method of the present invention involves the use of a culture medium which comprises preferably only compounds which can be administered intravenously, for instance, to mammalian, preferably human, subjects. Hence, in a preferred embodiment of the present invention, the culture medium of the present invention does not comprise compounds which would result in a supernatant which would be not intravenously administrable to mammalian, preferably human, subjects. Particularly preferred is the use of a Ringer's solution as culture medium, which may comprise further proteins like albumin and/or insulin as described above.

Before and/or during cultivating PBMCs in the cell culture medium of the present invention, the cells are preferably subjected to ionizing radiation. In addition to these stress inducing conditions the PBMCs can be subjected to further stress. Hence, according to a preferred embodiment of the present invention the PBMCs are subjected to one or more further stress inducing conditions before or during cultivation. Conditions causing stress to cells include among others heat, chemicals, radiation, hypoxia, osmotic pressure etc.

According to a preferred embodiment of the present invention the stress inducing conditions include hypoxia, ozone, heat (e.g. more than 2°C, preferably more than 5°C, more preferably more than 10°C, higher than the optimal cultivation temperature of PBMCs, i.e. 37°C), radiation (e.g. UV radiation), chemicals, osmotic pressure (i.e. osmotic conditions which are elevated at least 10% in comparison to osmotic conditions regularly occurring in a body fluid, in particular in blood) or combinations thereof.

According to another preferred embodiment of the present invention the PBMCs are subjected to an ionizing radiation at a dose of at least 10 Gy, preferably at least 20 Gy, more preferably at least 40 Gy, more preferably at least 50 Gy.

According to a preferred embodiment of the present invention the PBMCs are cultivated for at least 4 h, preferably for at least 6 h, more preferably for at least 12 h, before isolating its supernatant.

According to a preferred embodiment of the present invention the PCBMC cell culture comprises 1×10⁵ to 1×10⁸ PBMCs/ml, preferably 1×10⁶ to 1×10⁷ PBMCs/ml, more preferably 2×10⁶ to 5×10⁶ PBMCs/ml.

It turned out that the composition of the supernatant obtainable by cultivating PBMCs shows advantageous properties if a certain amount of PBMCs per ml cell culture medium is cultivated.

In order to increase the shelf-life of the supernatant obtainable with the method according to the present invention, the supernatant may be lyophilised. Methods for lyophilising such preparations are well known to the person skilled in the art. Prior its use the lyophilised preparation can be contacted with water or an aqueous solution comprising buffers, stabilizers, salts etc.

It turned surprisingly out that with the method of the present invention using the inventive culture medium supernatants of PBMCs can be obtained which show similar, the same or even better properties as supernatants obtained by cultivating PBMCs with cell culture media typically used for mammalian cell cultures (e.g. CellGro medium, Cellgro GMP DC medium, RPMI, DMEM, X-vivo and Ultraculture as used to obtain PBMC supernatants). The properties are measured using a potency assay as described in EP 3 729 079 B1, preferably potency assays involving the measurement of AP-1 promoter activities and/or the amount of phosphorylated HSP-27 as described therein.

A particularly preferred aspect of the present invention relates to a method for cultivating PBMCs comprising the step of incubating said cells in a culture medium comprising or consisting of Ringer's solution under an atmosphere comprising less than 1% carbon dioxide. The culture medium may further comprise a lactate, preferably sodium lactate. The supernatant of the PBMC culture can be isolated from the cells or cell debris after its cultivation.

Supernatants obtainable with the method of the present invention by cultivating PBMCs can be employed in the treatment and prevention of many diseases and disorders. For instance, these supernatants show beneficial effects in wound healing, in the treatment of effects caused by myocardial infarction or stroke and in the treatment and prevention of allergies. The presence of insulin in the culture medium has not only a beneficial effect on the cells to be cultivated but also in the supernatant obtained from such cell cultures. It is known that insulin shows immunomodulatory effects and may influence keratinocytes, fibroblasts and endothelial cells, thus, having positive effects on wound healing.

The present invention is further illustrated in the following examples, however, without being restricted thereto.

### EXAMPLES

### Materials and Methods

### PBMC isolation and generation of PBMC secretomes

PBMCs were obtained by Ficoll-Paque PLUS (GE Healthcare, USA)-assisted density gradient centrifugation and exposed to 60 Gy Caesium 137 gamma-irradiation (IBL 437C, Isotopen Diag-nostik CIS GmbH, Germany). Cells were adjusted to a concentration of 2.5×10⁷ cells/mL (equates to 25 U/ml) and cultured for 24 hours in different media as summarized in Table 1 under an atmosphere comprising approx. 5% CO₂ or approx. 0.04% CO₂ (atmospheric air). Cells and cellular debris were removed by centrifugation and supernatants were passed through a 0.2 µm filter.

**Table 1**

| **Medium No.** | **Medium composition** |
|---|---|
| 1 | CellGenix GMP DC medium (CellGenix GmbH, Germany) |
| 2 | Ringer's solution |
| 3 | Ringer's solution + 8 g/L human albumin |
| 4 | Ringer's solution + 8 g/L human albumin + 5 mg/L human insulin |

### HSP27 Phosphorylation Assay

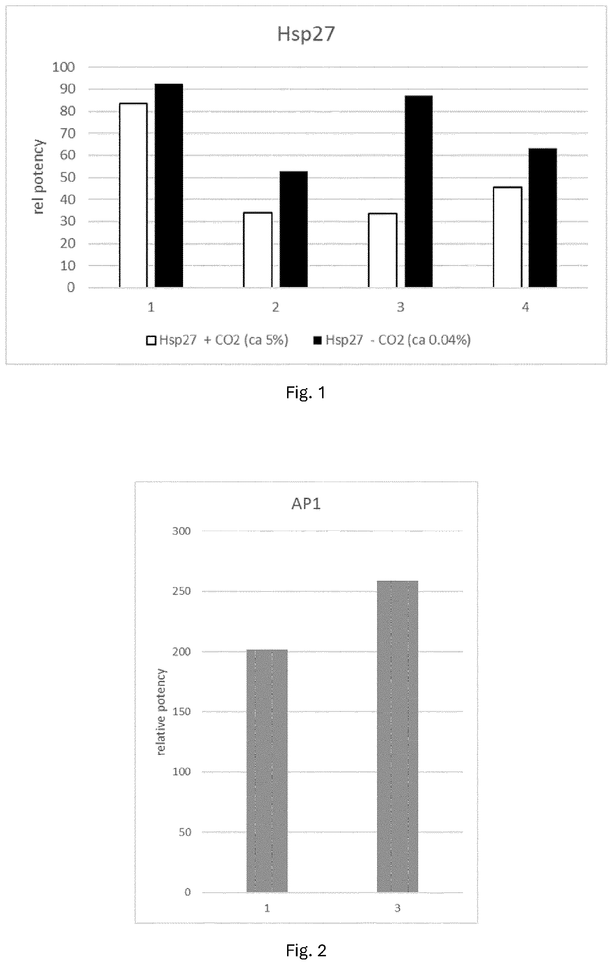

To detect HSP27 phosphorylation at residue Ser82, A549 cells were treated for 30 minutes, fixed, and permeabilized. Phosphorylation was assessed by sequential addition of an antibody detecting phosphorylated HSP27, followed by a peroxidase-conjugated anti-rabbit IgG antibody and chemiluminescent peroxidase substrate. The plates were read in the luminescence reader. The relative potency was calculated for the analyzed samples compared to the standard using the PLA software. For a detailed protocol see EP 3 729 079 B1. The results for the media of Table 1 are shown in Fig. 1.

### AP-1 Reporter Gene Assay

Human neuroblastoma SH-SY5Y cells containing an integrated expression cassette that encodes firefly luciferase under the transcriptional control of AP-1 were cultured in Ham's F12/MEM (50:50) supplemented with 2 mM L-glutamine, 1x MEM Non-essential Amino Acid Solution, 15% fetal bovine serum, and 1 ug/ml puromycin or DMEM/F12 + Glutamax supplemented with 10% fetal bovine serum and 1 ug/ml puromycin. To determine potency, SH-SY5Y AP-1 cells were seeded into 96-well plates (20,000 cells/well) and incubated overnight. After stimulation with the supernatant, luciferase substrate was added to all wells and plates were read in a luminescence reader. For a detailed protocol see EP 3 729 079 B1. The results for media 1 and 3 of Table 1 are shown in Fig. 2.

### Cytokine Assay

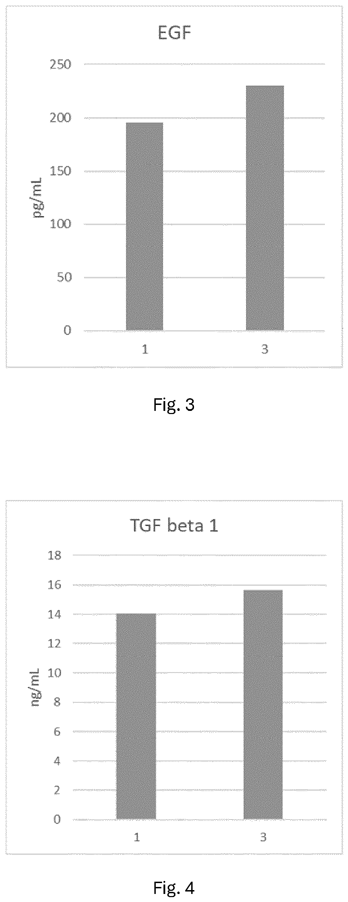

The concentrations of EGF and TGF beta 1 were measured by enzyme-linked immunosorbent assays as described in Laggner M et al (Stem Cell Res Ther. 2020 Jan 3;11(1):9. doi: 10.1186/s13287-019-1524-2). The results for media 1 and 3 of Table 1 are shown in Figs. 3 and 4.

### Sprouting Test

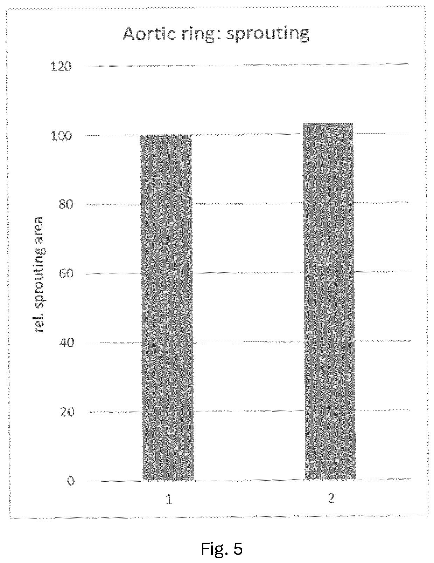

Pro-angiogenic potential of the supernatant was functionally tested by a sprouting assay using murine thoracic aortas embedded in a hydrogel (see Laggner M et al. (Stem Cell Res Ther. 2020 Jan 3;11(1):9. doi: 10.1186/s13287-019-1524-2)). Aortic rings were sandwiched in fibrin matrices and cultured with secretome at a concentration of 4 U/mL for five to seven days. The results for media 1 and 2 of Table 1 are shown in Fig. 5.

### Summary of Results

The results obtained using the assays described above show that culture media based on Ringer's solution give similar or even better results compared to a medium which is usually used to obtain supernatants of PBMCs using an atmosphere comprising atmospheric carbon dioxide concentrations.

## Claims

1. Method for cultivating mammalian cells comprising the step of incubating mammalian cells in a culture medium comprising at least one sodium salt, at least one potassium salt and at least calcium salt under an atmosphere comprising less than 1% carbon dioxide.

2. Method according to claim 1, wherein the atmosphere comprises less than 0.5%, preferably less than 0.2%, more preferably less than 0.1%, more preferably less than 0.05%, carbon dioxide.

3. Method according to claim 1 or 2, wherein the atmosphere comprises oxygen and/or nitrogen.

4. Method according to any of claims 1 to 3, wherein the culture medium comprises 50 to 200 mmol/L, preferably 100 to 150 mmol/L, more preferably 125 to 135 mmol/L, more preferably 130 to 132 mmol/L, more preferably around 131 mmol/L, sodium ions.

5. Method according to any of claims 1 to 4, wherein the at least one sodium salt is sodium chloride.

6. Method according to any of claims 1 to 5, wherein the culture medium comprises 2 to 8 mmol/L, preferably 3 to 6 mmol/L, more preferably 4 to 6 mmol/L, more preferably 5 to 6 mmol/L, more preferably around 5.36 mmol/L, potassium ions.

7. Method according to any of claims 1 to 6, wherein the at least one potassium salt is potassium chloride.

8. Method according to any of claims 1 to 7, wherein the culture medium comprises 0.5 to 3 mmol/L, preferably 1 to 2 mmol/L, more preferably 1.5 to 2 mmol/L, more preferably around 1.84 mmol/L, calcium ions.

9. Method according to any of claims 1 to 8, wherein the at least one calcium salt is calcium chloride.

10. Method according to any one of claims 1 to 9, wherein the culture medium is Ringer's solution.

11. Method according to any of claims 1 to 10, wherein the culture medium comprises further at least one salt of a C₂ to C₅ carbonic acid, wherein the C₂ to C₅ carbonic acid is preferably lactate, more preferably L-lactate, and/or wherein the at least one salt of a C₂ to C₅ carbonic acid is an alkaline salt or an alkaline earth salt, preferably a sodium salt.

12. Method according to any one of claims 1 to 11, wherein the culture medium comprises at least one polypeptide, preferably at least one blood polypeptide, more preferably at least one polypeptide selected from the group consisting of albumin, preferably serum albumin, and insulin.

13. Method according to claim 12, wherein the culture medium comprises 1 mg/L to 50 g/L, preferably 2 mg/L to 40 g/L, more preferably 4 mg/L to 30 g/L, more preferably 1 g/L to 20 g/L, more preferably 2 g/L to 10 g/L, of the at least one polypeptide.

14. Method according to any one of claims 1 to 13, wherein the culture medium comprises 1 to 50 g/L, preferably 2 to 40 g/L, more preferably 4 to 30 g/L, more preferably 5 to 20 g/L, more preferably 6 to 10 g/L, albumin, preferably serum albumin, and/or 1 to 20 mg/L, preferably 2 to 15 mg/L, more preferably 3 to 10 mg/L, more preferably 4 to 6 mg/L, insulin.

15. Method according to any one of claims 1 to 14, wherein the cells are peripheral blood mononuclear cells (PBMCs), more preferably monocytes, T cells, B cells and/or NK cells.
